# EUROPEAN PATENT SPECIFICATION

(11) **EP 0 434 960 B1**
(45) Date of publication and mention of the grant of the patent: **18.09.1996**
(21) Application number: 90121905.5
(22) Date of filing: 15.11.1990
(51) Int. Cl.: C07H 15/252, A61K 31/71, C07C 43/313

(54) **Chiral 1,5-diiodo-2-methoxy or benzyloxy intermediates**
Chirale 1,5-diiodo-2-methoxy oder benzyloxy Zwischenprodukte
1,5-diiodo-2-methoxy ou benzyloxy intermédiates chiraux

(30) Priority: 19.12.1989 GB 8928654; 03.04.1990 GB 9007513
(43) Date of publication of application: 03.07.1991
(73) Proprietor: PHARMACIA S.p.A., 20152 Milano (IT)
(72) Inventor: Faiardi, Daniela, I-27100 Pavia (IT); Bargiotti, Alberto, I-20100 Milan (IT); Grandi, Maria, I-20100 Milan (IT); Suarato, Antonino, I-20158 Milan (IT)
(74) Representative: Giambrocono, Alfonso, Dr. Ing.

(56) References cited:
- EP-A- 0 188 293
- GB-A- 2 172 594
- US-A- 4 374 980
- J. Med. Chem. 1982, 25, 18-24
- J. Med. Chem. 1984, 27, 638-45
- J. Antibiot, 1982, 35, 117-8
- Total Synthesis of natural Products : The Chiron Approach S. Hanessioon, Pergamon, Oxford UK 1983

## Description

The invention provides new chiral intermediates of general formula C 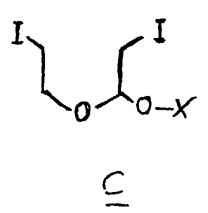 wherein X represents a methyl group or a benzyl residue -CH₂C₆H₅.

The compounds which may have a (S) or (R) configuration at carbon atom C-2" of the morpholino ring are useful intermediates for preparing anthracycline glycoside antibiotics.

The invention provides a process for the preparation of optically pure diiodo compounds C, starting from sugar precursors such as the compound of general formula S derived from L-arabinose: 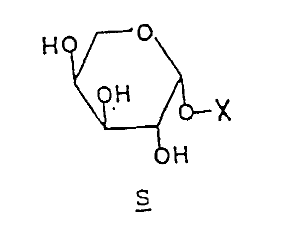 wherein X is as defined above. This process comprises:
(a) subjecting to periodate oxidation a compound of formula S^{·} : 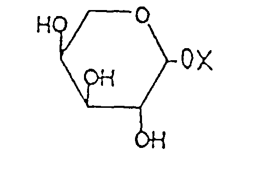 wherein X is as defined above;
(b) reducing the thus-obtained dialdehyde derivative of formula D·· : 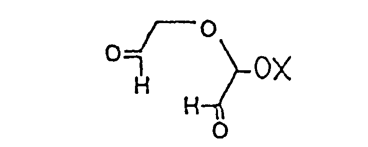 wherein X is as defined above;
(c) sulfonating the thus-obtained dihydroxy derivative of formula E· : 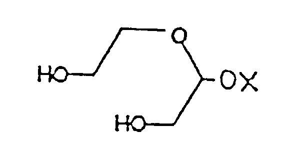 wherein X is as defined above; and
(d) iodinating the sulfonated derivative thus obtained.

In order to prepare the diiodo compounds C, 1-substituted sugars S· , prepared following standard procedures described in "Methods on Carbohydrate Chemistry" Acad. Press., Vol 1, (1962), are first transformed into dialdehyde derivatives D· . Generally, D- or L-arabinose is employed as a starting material. This is reacted with an alcohol X-OH thereby to form the compound of formula S·· . The dialdehyde derivatives can be obtained by using periodate oxidation in water, then reduced to 1,5-dihydroxy-2-alkoxy or -benzyloxy-3-oxa-pentane E· by using reducing agents such as sodium borohydride or sodium cyanoborohydride at pH 6.5 in a mixture of water and methanol.

The resultant dihydro compounds E· are sulfonated at the 1- and 5-hydroxyl groups, typically by using p-toluensulfonyl chloride in pyridine at 4°C to give the sulfonyl esters of formula F· from which the diiodo derivatives C are obtained upon treatment with sodium or potassium iodide in aprotic solvent such as methylethylketone at 85°C from one to two days. The sequence of these reactions do not affect the chirality at C-2 of the diiodo derivatives C which is the same of the starting sugars S.

A preferred embodiment of a process according to the invention is illustrated by the following reaction Scheme:

### Reaction Scheme

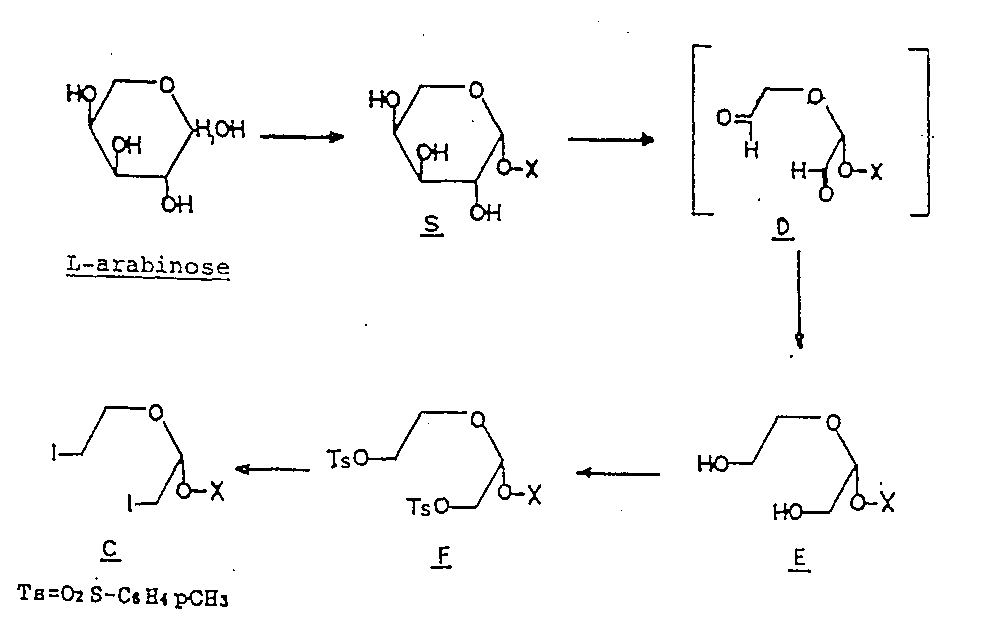

The following Examples illustrate the invention.

### Example 1

### Preparation of 1, 5-di(p-toluensulphonyl),oxy-2(s)-benzyloxy-3-oxa-pentane (F1)

L-arabinose (3 g, 0.022 mole) and benzyl alcohol (15 ml) were heated under stirring in presence of Dowex 50Wx2 (2 g) in acidic form. After four hours, the mixture was cooled and filtered. The solvent was removed under reduced pressure and l-benzyl-β-L-arabinopyranoside (S1, 3.5 g) was recovered from acetone. TLC on Kieselgel Plate F₂₅₄ (Merck), eluting system methylene chloride/methanol/water (120/20/2 by volume) Rur: Rf=0.47. [α]_{D}=+215° (c=1% water).

1-benzyl-β-L-arabinopyranoside (S1, 3.48 g, 0.0145 mole) was dissolved in water (100 ml) and treated with sodium periodate (5.6 g, 0.026 mole) at 0°C for two hours. Then barium chloride was added and the mixture was brought to pH 7 with barium carbonate, filtered off and washed with water. The aqueous solution was concentrated under reduced pressure to a syrup and extracted with acetonitrile (50 ml). The organic phase was diluted with a mixture of methanol (20 ml) and water (10 ml) and treated with sodium cyanoborohydride (0.3 g) dissolved in water (5 ml). After 15 minutes the mixture was brought to pH 7 by adding Dowex 50Wx2 and filtered. The solvents were removed under reduced pressure to give 1,5-dihydroxy-2(S)-benzyloxy-3-oxa-pentane (E1., 2.6g, yield 85%). TLC on Kieselgel Plate-F₂₅₄ (Merck), eluting system methylene chloride/methanol (10/1 by volume) Rur: Rf=0.28. Compound E1 (2.6 g) was dissolved in dry pyridine and added with p-toluensulfonylchloride (6.67 g). The mixture was kept at 0°C overnight, then poured into ice-water and extracted with methylene chloride. The organic phase was washed with water, separated off, dryied over anhydrous sodium sulphate and filtered off. The solvent was removed under reduced pressure to afford 1,5-di(p-toluensulphonyl)oxy-2(S)-benzyloxy-3-oxa-pentane (F1, 4.3 g, yield 68%). TLC on Kieselgel Plate F₂₅₄ (Merck), eluting system methylene chloride/acetone (98/2 by volume) R_{f}=0.55
¹HNMR (CDCl₃, 400 MHz) δ:
2.42 (s, 6H, two CH₃Ts); 3.65, 3.69 (two dt, J=4.7, 11.7Hz, 2H, TsOCH₂CH₂O); 3.94 (two, dd, J=5.3, 10.5Hz, 2H, OCH-CH₂-OTs); 4.08 (t, J=4.7Hz, 2H, Ts-O-CH₂CH₂-O); 4.46, 4.56 (two, d, J=11.7Hz, 2H, OCH₂-Ph); 4.72 (t, J=5.3Hz, 1H, O-CH-CH₂-OTs); 7.2-7.8 (m, 13H, aromatics).

### Example 2

### Preparation of 1,5-diiodo-2(S)-benzyloxy-3-oxa-pentane (C1).

Compound F1 (4.3 g, 8.3 mmole), prepared as described in Example 1, was dissolved in methylethylketone (50 ml) and added with sodium iodide (7.4 g, 49 mmole). The mixture was kept at 95°C for twentyfour hours. After that, the solvent was removed under reduced pressure and the residue was extracted with n-hexane. The organic phase was concentrated to a syrup to give the title compound C1 (3.5 g, yield 90%). TLC on Kieselgel Plate F₂₅₄ (Merck), eluting system n-hexane/ethyl acetate (10/0.5 by volume) R_{f}=0.34
¹HNMR (CDCl₃, 400 MHz) δ :
3.27 (t, J=6.8Hz, 2H, J-CH₂CH₂-O); 3.30 (d, J=5.5Hz, 2H, O-CH-CH₂-J); 3.84 (m, 2H, J-CH₂,CH₂-O); 4.63, 4.74 (two d, J=11.7Hz, 2H, O-CH₂Ph); 4.81 (t, J=5.5Hz, 1H, O-CH-CH₂-J); 7.3-7.5 (m, 5H, aromatics).

### Example 3

### Preparation of 3'-deamino-3'[2(s)-benzyloxy-4-morpholinyl] doxorubicin (A1)

To a solution of doxorubicin hydrochloride (0.5 g, 0.86 mmole) in dry dimethylformamide (20 ml) was added 1,5-diiodo-2(S)-benzyloxy-3-oxa-pentane (C1, 3.5 g, 7.54 mmole) and dry triethylamine (3.6 ml, 2.6 mmole). The mixture was kept at room temperature for 36 hours, then was poured in water and extracted with methylene chloride. After standard work-up, the crude product was purified on silicic acid column using as eluting system a mixture of methylene chloride/methanol (97/5 by volume), to give, after treatment with methanolic anhydrous hydrogen chloride, the title compound A1 (0.3 g, yield 46%) as hydrochloride salt. TLC on Kieselgel Plate F₂₅₄ (Merck), eluting system methylene chloride/methanol (10/1 by volume) R_{f}=0.6.
FD-MS: m/e 756 (M+)
¹HNMR of free base (CDCl₃, 200 MHz) δ:
1.37 (d, J=6.6Hz, 3H, 5'-CH₃); 1.76 (m, 2H, 2'-CH₂); 2.14 (dd, J=3.9, 14.8Hz, 1H, 8ax-H); 2.2-2.7 (m, 6H, CH₂-N-CH₂, 8eq-H, 3'-H); 3.00 (d, J=18.8Hz, 1H, 10ax-H); 3.55, 4.00 (two m, 2H, O-CH₂CH₂N); 3.68 (s, 1H, 4'-H); 3.94 (q, J=6.6Hz, 1H, 5'-H); 4.08 (s, 3H, OCH₃); 4.51, 4.77( two d, J=12.1Hz, 2H, OCH₂Ph); 4.65 (dd, J=2.6, 4.0Hz, 1H, OCH(OCH₂Ph)CH₂N); 4.71 (s, 1H, COCH₂OH); 5.28 (dd, J=2.4, 3.8Hz, 1H, 7-H); 5.54 (m, 1H, 1'-H); 7.2-8.1 (m, 8H, aromatic H's); 13.22 (s, 1H, 11-OH); 13.95 (s, 1H, 6-OH).

### Example 4

### Preparation of 1,5-diiodo-2(S)-ethoxy-3-oxa-pentane (C2).

The title compound C2 was prepared starting from L-arabinose (3 g) following sequential reactions as described in Example 1 and Example 2.
1-ethyl-α-L-arabinopyranoside (S2): [α]_{D}= +233.5° (c=1% water 1,5-dihydroxy-2(S)-ethoxy-3-oxa-pentane (E2): TLC on Kieselgel Plate F₂₅₄ (Merck) eluting system methylene chloride/methanol (10/1 by volume) R_{f}=0.33. 1,5-di(p-toluensulphonyl)oxy-2(S)-ethoxy-3-oxa-pentane (F2): TLC on Kieselgel Plate F₂₅₄ (Merck), eluting system methylene chloride/acetone (98/2 by volume) R_{f}=0.56
¹HNMR (CDCl₃, 200MHz) δ:
1.11 (t, J=7.0Hz, 3H, OCH₂CH₃); 2.43 (s, 6H, two CH₃-Ts); 3.44, 3.58 (two dq, J=7.0, 9.4Hz, 2H, OCH₂CH₃); 3.68 (m, 2H, O-CH₂-CH₂-OTs); 3.89 (d, J=5.4, 2H, O-CH-CH₂-OTs); 4.10 (t, J=4.8Hz, 2H, OCH₂-CH₂OTs); 4.61 (t, J=5.4Hz, 1H, O-CH-CH₂OTs); 7.3-7.8 (m, 8H, aromatic H's). 1,5-diiodo-2(S)-ethoxy-3-oxa-pentane (C2): TLC on Kieselgel Plate F₂₅₄ (Merck), eluting system n-hexane/ethyl acetate (10/0.5 by volume) R_{f}=0.37
¹HNMR (CDCl₃, 200MHz) δ:
1.24 (t, 7.0Hz, 3H, OCH₂CH₃); 3.23 (d, J=5.6Hz, 2H, J-CH₂CH-O); 3.27 (t, J=6.8Hz, 2H, J-CH₂CH₂-O); 3.58, 3.77 (two dq, J=7.0, 9.3Hz, 2H, OCH₂CH₃); 3.78, 3.87 (two dt, J=6.8, 10.6Hz, 2H, J-CH₂CH₂-O); 4.70 (t, J=5.6Hz, 1H, O-CH-CH₂-J).

### Example 5

### Preparation of 3'-deamino-3'[2(S)-ethoxy-4-morpholinyl] doxorubicin (A2)

Doxorubicin hydrochloride (0.5 g) was reacted with compound C2 (3 g) following the same procedure reported in Example 3 to give the title compound A2 (0.28 g) as hydrochloride salt.
TLC on Kieselgel Plate F₂₅₄ (Merck), eluting system methylene chloride/methanol (10/1 by volume) R_{f}=0.58
FD-MS: m/e 694 (M+)
¹HNMR as free base (CDCl₃, 200MHz) δ:
1.16 (t,J=7.0Hz, 3H, OCH₂,CH₃); 1.36 (d, J=6.4Hz, 3H, 5'-CH₃); 1.7-1.8 (m, 2H, 2'-CH₂,); 2.16 (dd, J=4.1, 14.7Hz, 1H, 8ax-H); 2.3-2.6 (m, 6H, CH₂,-N-CH₂);, 8eq-H, 3'-H); 3.02 (d, J=18.8Hz, 1H, 10ax-H); 3.26 (dd, 1.9, 18.8Hz, 1H, 10eq-H); 3.46, 3.75 (two dq, J=7.0, 9.8Hz, 2H, OCH₂CH₃); 3.53, 3.93 (two m, 2H, OCH₂CH₂,N); 3.68 (s, 1H, 4'-H); 3.93 (q, J=6.4Hz, 1H, 5'-H); 4.08 (s, 3H, OCH₃); 4.56 (dd, J=2.3, 4.7Hz, 1H, OCH(OCH₂CH₃)CH₂N); 4.72 (s, 1H, 9-OH); 4.74 (s, 2H, COCH₂OH); 5.30 (m, 1H, 7-H); 5.55 (m, 1H, 1'-H); 7.3-8.1 (m, 3H, aromatic H's); 13.25 (s, 1H, 11-OH); 13.97 (s, 1H, 6-OH)

### Example 6

### Preparation of 1-5-diiodo-2(R)-isopropyloxy-3-oxa-pentane (C3)

The title compound C3 was prepared starting from L-arabinose (3 g) following sequential reactions as described in Example 1 and Example 2.
1-isopropyl-β-L-arabinopyranoside (S3): [α]_{D}= +225° (water) 1,5-dihydroxy-2(R)-isopropyloxy-3-oxa-pentane (E3):
TLC on Kieselgel Plate F₂₅₄ (Merck), eluting system methylene chloride/methanol (10/1 by volume) R_{f}=0.36 1,5-di(p-toluensulphonyl)oxy-2(R)-isopropyloxy-3-oxa-pentane (F3) TLC on Kieselgel Plate F₂₅₄ (Merck), eluting system methylene chloride/acetone (95/2 by volume) R_{f}=0.55
¹HNMR (CDCl₃, 200MHz) δ:
1.05, 1.10 (two d, J=6.2Hz, 6H, CH(CH₃)₂); 2.42 two s, 6H, CH₃-Ts); 3.64 (two m, 2H, Ts-O-CH₂CH₂-O); 3.76 (m, 1H, CH(CH₃)₂); 3.84 (m, 2H, O-CH-CH₂-OTs); 4.08 (t, J=5.6Hz, 2H, Ts-O-CH₂CH₂-O); 7.3-7.8 (m, 8H, aromatic H's). 1,5-diiodo-2(R)-isopropyloxy-3-oxa-pentane (C3) TLC on Kieselgel Plate F₂₅₄ (Merck), eluting system n-hexane/ethyl acetate (10/0.5 by volume) R_{f}=0.40
¹HNMR (CDCl₃, 200MHz) δ:
1.20, 1.22 (two d, J=6.4Hz, 6H, CH(CH₃)₂); 3.24 (d, J=5.6Hz, 2H, O-CH-CH₂-J); 3.28 (t, J=6.7Hz, 2H, J-CH₂CH₂-O); 3.6-3.8 (m, 2H, J-CH₂CH₂-O); 3.94 (m, 1H, CH(CH₃)₂); 4.76 (t, J=5.6Hz, 1H, O-CH-CH₂-J).

### Example 7

### Preparation of 3'-deamino-3-[2(R)-isopropyloxyl-4-morpholinyl] doxorubicin (A3)

Doxorubicin hydrochloride (0.5 g) was reacted with compound C3 (3.2 g) following the same procedure reported in Example 3 to give the title compound A3 (0.21 g) as hydrochloride salt.
TLC on Kieselgel Plate F₂₅₄ (Merck), eluting system methylene chloride/methanol (10/1 by volume) R_{f}=0.55
FD-MS: m/e 708 (M+)
¹HNMR as free base (CDCl₃, 200MHz) δ:
1.09, 1.16 (two d, J=6.0Hz, 6H, CH(CH₃)₂); 1.36 (d, J=6.6Hz, 3H, 5'-CH₃); 1.80 (m, 2H, 2'-CH₂); 2.15 (dd, J=4.0, 14.9Hz, 1H, 8ax-H); 2.3-2.8 (m, 6H, CH₂NCH₂, 8eq-H, 3'-H); 2.97 (d, J=18.8Hz, 1H; 10ax-H); 3.26 (d, J=18.8Hz, 1H, 10eq-H); 3.54 (m, 1H, O-CH(H)CH₂N); 3.74 (s, 1H, 4'-H); 3.81-4.1 (m, 3H, OCH(H)CH₂N, 5'-H, OCH(CH₃)₂); 4.08 (s, 3H, OCH₃); 4.66 (s, 1H, 9-OH); 4.68 (dd, J=2.2, 4.9Hz, 1H, OCH[OCH(CH₃)₂]CH₂N); 4.75 (s, 2H, COCH₂OH); 5.28 (m, 1H, 7-H); 5.55 (m, 1H, 1'-H); 7.3-7.8 (m, 3H, aromatic H's); 13.24 (s, 1H, 11-OH); 13.97 (s, 1H, 6-OH).

### _{Example 8:}

### 1,5-dihydroxy-2(S)-methoxy-3-oxa-pentane (E4)

1,5-dioxo-2(S)-methoxy-3-oxa-pentane (D4 ) (1.5 g, 11mmole), prepared as described in "Methods on Carbohydrate Chemistry" Acad.Press., Vol.1, 445, (1962), was dissolved in a mixture of water (10 ml) and methanol (10 ml) and treated with, sodiumborohydride (0,1g) dissolved in water (2 ml). After 20 minutes the solution was brought to pH 7 with an acidic resin Dowex 50WX2, filtered off and the solvent was removed under reduced pressure to give 1.4 g (Yield 93%) of the title compound, TLC on Kieselgel Plate F₂₅₄ (Merck), eluting system methylene chloride:methanol (10:1 by volume) brown spot at R_{f}=0.24 after heating the TLC plate previously sprayed with sulforic acid.
¹HNMR (200 MHz, DMSO-d₆) δ:
3.26 (s, 3H, OCH₃); 3.4-3.6 (m, 6H, -CH₂-CH₂-O-CH-CH₂-); 4.37 (t, J=5.4 Hz, 1H, O-CH-O); 4.40 (bm, 2H, HO-CH₂CH₂, CH₂CH₂₋OH)

### Example 9

### 1,5-di(p-toluensulfonyl)oxy-2(S)-methoxy-3-oxa-pentane (F4 )

1,5-dihydroxy-2(S)-methoxy-3-oxa-pentane (E4) (1.4 g, 10.3 mmole), prepared as described in Example 8, was dissolved in dry pyridine (10 ml) and treated at 0°C with p-toluensulfonylchloride (6.4 g, 0,034 mole). The mixture was kept at 4°C overnight, then was poured into an ice-water mixture and finally extracted with methylene chloride. The organic phase was washed with water, separated off, dried over anhydrous sodium sulphate, filtered off. The solvent was removed under reduced pressure. The crude material was chromatographed on a silicic acid column using methylene chloride as eluting agent to give 2.8 g (yield 62%) of pure title derivative. TLC on Kieselgel Plate F₂₅₄ (Merck), eluting system methylene chloride:acetone (95:5 by volume) brown spot at R_{f}=0.55 after heating the TLC plate previously sprayed with sulforic acid.
¹HNMR (200 MHz, CDCl₃) δ:
2.44 (s, 6H, CH₃-Ph); 3.27 (s, 3H, OCH₃); 3.69 (m, 2H, SO₂OCH₂CH₂-O); 3.90 (m, 2H, SO₂OCH₂-CH-O); 4.11 (m, 2H, SO₂OCH₂CH₂-O); 4.56 (t, J=5.3Hz, 1H,-O-CH-CH₂); 7.3-7.8 (m, 8H, aromatic H's).

### Example 10

### Preparation of 1,5-diiodo-2(S)methoxy-3-oxa-pentane (C4 )

1,5-di(p-toluensulfonyl)oxy-2(S)-methoxy-3-oxa-pentane (F4 ) (1.6 g, 3.6 mmole), prepared as described in Example 9, was dissolved in methylethylketone (30 ml) and treated with sodium iodide (3.04 g, 20.2 mmole) at 85°C for two days. After that, the solvent was removed in vacuo and the residue was added with n-hexane (50 ml) and water. The organic phase was separated off, dried over anhydrous sodium sulphate, filtered off. The solvent was removed under reduced pressure to give 1,5-diiodo-2(S)-methoxy-3-oxa-pentane (C4 ) (1.2 g, yield 86%). TLC on Kieselgel Plate F₂₅₄ (Merck), eluting system methylene chloride, brown spot at R_{f}=0.54 after heating the TLC plate previously sprayed with sulforic acid.
¹HNMR (200 MHz, CDCl₃) δ:
3.15 (m, 4H, J-CH₂CH₂-OCH-CH₂-J); 3.40 (s, 3H, OCH₃); 3.80 (m, 2H, J-CH₂CH₂-O); 4.62 (t, J=5.6 Hz, 1H, O-CH-CH₂).

### Example 11

### Preparation of 3'-deamino-[2"(S)-methoxy-4"-morpholinyl]-doxorubicin (A4)

To a solution of doxorubicin hydrochloride (80 mg, 0.138 mmole) dissolved in dry dimethylformamide (4 ml) was added 1,5-diiodo-2(S)-methoxy-3-oxa-pentane ( C4 , 0.8 g, 2.06 mmole) and triethylamine (0.056 ml, 0.388 mmole). The reaction mixture was kept at room temperature under stirring for 36 hrs, then was poured in water and extracted with methylene chloride. After standard work up, the crude product was purified on silicic acid column using as eluting system amixture of methylene chloride methanol (97.5 : 2.5 by volume), to give, after treatment with methanolic anhydrous hydrogen chloride, 40 mg (yield 45%) of the title compound as hydrochloride salt. TLC on Kieselgel Plate F₂₅₄ (Merck), eluting system methylene chloride:methanol (19:1 by volume) R_{f}=0.15
Free base: ¹HNMR (400 MHz, CDCl₃), δ:
13.98 (s, 1H, 6-OH); 13.27 (s, 1H, 11-OH); 8.03 (dd, J=1.1, 7.7Hz, 1H, 1-H); 7.78 (dd, J=7.7, 8.6Hz, 1H, 2-H); 7.39 (dd, J=1.1, 8.6Hz, 1H, 3-H); 5.55 (m, 1H, 1'-H); 5.30 (dd, J=2.1, 4.1Hz, 1H, 7-H); 4.74 (d, J=4.5Hz, 14-CH₂OH); 4.74 (S, 1H, 9-OH); 4.49 [dd, J=2.6, 4.1Hz, 1H, NCH₂-CH(OCH₃)O]; 4.08 (s, 3H, 4-OCH₃); 3.94 (q, J=6.6Hz, 1H, 5'-H); 3.93 [m, 1H, NCH₂CH(H)O]; 3.67 (m, 1H, 4'-H); 3.54 [m, 1H, NCH₂CH(H)O]; 3.38 [s, 3H, NCH₂CH-OCH₃]; 3.27 (dd, J=19, 18.8Hz, 1H, 10-Heq); 3.04 (d, J=18.8Hz, 1H, 10-Hax); 3.00 (t, J=4.5Hz, 1H, CH₂OH); 2.60 [dd, J=4.l, 11.4Hz, 1H, NCH(H)CHOCH₃]; 2.6-2.5 (m, 3H, NCH(H)CHOCH₃, NCH₂CH₂O); 2.4-2:3 (m, 2H, 8-Heq, 3'-H); 2.15 (dd, J=4.1, 14.7Hz, 8-Hax); 1.76 (m, 2H, 2'-CH₂); 1.26 (d, J=6.6Hz, 3H, 5'-CH₃).

### Example 12

### Preparation of 1,5-dihydroxy-2(R)-methoxy-3-oxa-pentane (E5)

The title compound was prepared as described in Example 8 starting from 1,5-dioxo-2(R)-methoxy-3-oxa-pentane (D5 ) which in turn can be prepared as described in "Methods on Carbohydrate Chemistry" Acad.Press., Vol.1, 445, (1962). TLC on Kieselgel Plate F₂₅₄ (Merck), eluting system methylene chloride:methanol, brown spot at R_{f}=0.24 after heating the TLC plate previously sprayed with sulforic acid.

### Example 13

### Preparation of 1,5-di(p-toluensulfonyl)oxy-2(R)-methoxy-3-oxa-pentane (F5 )

1,5-dihydroxy-2(R)-methoxy-3-oxa-pentane (E5), prepared as described in Example 12, was transformed into the title compound F5 following the same procedure reported in Example 9. TLC on Kieselgel Plate F₂₅₄ (Merck), eluting system methylene chloride: acetone (95:5 by volume) brown spot at R_{f}=0.55 after heating the TLC plate previously sprayed with sulforic acid.

### Example 14

### Preparation of 1,5-diiodo-2(R)-methoxy-3-oxa-pentane ( C5 )

1,5-di(p-toluensulfonyl)oxy-2(R)-methoxy-3-oxa-pentane ( F5 ), prepared as described in Example 13, was converted into the diiodo derivative C5 following the procedure described in Example 10, TLC on Kieselgel Plate F₂₅₄ (merck), eluting methylene chloride, system brown spot at R_{f}=0.54 after heating the TLC plate previously sprayed with sulfuric acid.

### Example 15

### Preparation of

### 3'-deamino-[2"(R)-methoxy-4"-morpholinyl]-doxorubicin (A5)

The title compound was prepared by condensing doxorubicin hydrochloride with 1,5-diiodo-2(R)-methoxy-3-oxa-pentane (C5), prepared as above described, following the procedure reported in Example 11.
TLC on Kieselgel Plate F₂₅₄ (Merck), eluting system methylene chloride:methanol (19:1 by volume) R_{f}=0.13
Free base: ¹HNMR (400 MHz, CDCl₃) δ:
13.97.(s, 1H, 6-OH);.13.25 (s, 1H, 11-OH); 8.03)(dd,J=1,1, 7.7Hz, 1H, 1-H); 7.78 (dd, J=7.6, 7.7Hz; 1H, 2-H); 7.39 (dd, J=1.1, 7.6Hz,1H, 3-H); 5.53 (d, J=3.4Hz, 1H, 1'-H); 5.29 (dd, J=2.5,- 4.1Hz, 1H, 7-H); 4.75 (s, 2H, 14-CH₂OH); 4.71 (s, 1H, 9-OH); 4.46 [dd, J=2.6, 4.7Hz, 1H, NCH₂-CH(OCH₃)O]; 4.08 (s, 3H, 4-OCH₃); 3.93 (q, J=6.6Hz, 1H, 5'-H); 3.92 [m, 1H, NCH₂CH(H)O]; 3.70 (m, 1H, .4'-H); 3.56 [m, 1H, NCH₂CH(H)O]; 3.40 [s, 3H, NCH₂CH-OCH₃]; 3.26 (dd, J=19, 19.9Hz, 1H, 10-Heq); 3.03 (d, J=19.9Hz, 1H, 10-Hax); 2.66 [dd, J=2.6, 11.4Hz, 1H, NCH(H)CHOCH₃]; 2.53 (m, 1H, NCH(H)CH₂O); 2.5-2.3 (m, 4H, NCH(H)CH₂O, NCH(H)CHOCH₃, 8-Heq, 3'-H); 2.15 (dd, J=4.1, 14.7Hz, 8-Hax); 1.8-1.7 (m, 2H, 2'-CH₂); 1.36 (d, J=6.6Hz, 3H, 5'-CH₃).

## Claims

1. A diiodo compound of formula C: 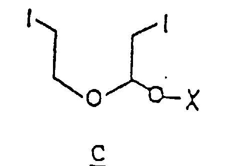 wherein X is a methyl or benzyl group.

2. A process for the preparation of a diiodo compound of formula C as defined in claim 1, which process comprises:
(a) subjecting to periodate oxidation a compound of formula S·· : 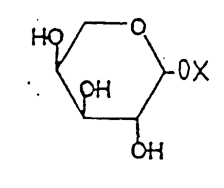 wherein X is as defined in claim 1;
(b) reducing the thus-obtained dialdehyde derivative of formula D·: 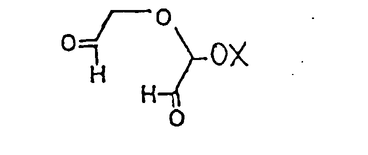 wherein X is as defined above;
(c) sulfonating the thus-obtained dihydroxy derivative of formula E·: 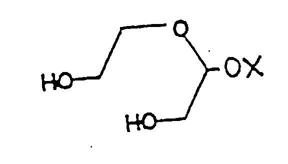 wherein X is as defined above; and
(d) iodinating the sulfonated derivative thus obtained.

3. A process according to claim 2, wherein D- or L-arabinose is reacted with an alcohol X-OH, wherein X is as defined in claim 1, thereby to form the compound of formula S··.

4. A process according to claim 2, wherein the dihydroxy derivative of formula E· is reacted with p-toluenesulfonyl chloride in step (c).

## Patentansprüche

1. Dijod Verbindung mit der Formel C: 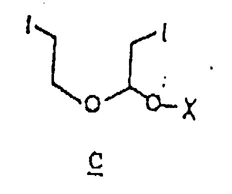 in der:
X eine Methyl oder Benzyl Gruppe ist.

2. Verfahren zur Herstellung einer Dijod Verbindung der Formel C, wie in Anspruch 1 definiert, wobei das Verfahren umfasst
(a) Oxidierung durch Periodat einer Verbindung der Formel S: 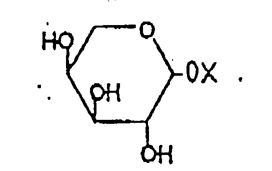 in der
X wie in Anspruch 1 definiert ist;
(b) Reduktion des auf diese Weise erhaltenen Dialdehydederivates der Formel D: 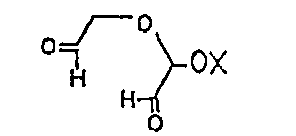 worin X wie oben definiert ist;
(c) Sulfurierung des auf diese Weise erhaltenen Dihydroxyderivates der Formel E: 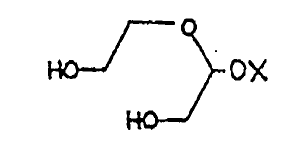 worin X wie oben definiert ist; und
(d) Jodisierung des auf diese Weise erhaltenen sulfonierten Derivates.

3. Verfahren nach Anspruch 2, worin D oder L Arabinose mit einem Alkohol X-OH reagiert,worin X wie in Anspruch 1 definiert ist, um die Verbindung der Formel S herzustellen.

4. Verfahren nach Anspruch 2, worin das Dihydroxyderivat der Formel E mit p-Toluenesulfonylchlorid nach Schritt (c) reagiert.

## Revendications

1. Composè diiode représenté par la formule C 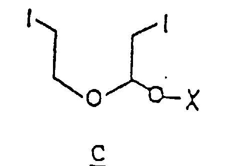 dans laquelle X représente un groupe methyl ou benzylique

2. Procédé de fabrication d'un composé diiode représenté par la formule C, tel que défini à la revendication 1, lequel procédè consiste à:
(a) soumettre à oxydation par periodate un composè
représenté par la formule S: 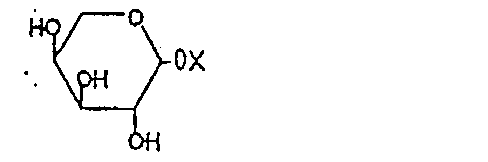 dans laquelle X est tel que défini à la revendication 1;
(b) reduire le dérivé dialdehyde, tel que obtenu représenté
par la formule D: 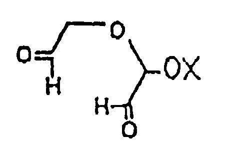 dans laquelle X est tel que dèfini ci-dessus
(c) sulfoner le dérivé dihydroxy tel que obtenu représenté par la formule E: 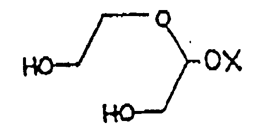 dans laquelle X est tel que défini ci-dessus; et
(d) iodurer le dérivé sulfoné tel que obtenu.

3. Procédé selon la revendication 2, dans lequel D-ou L arabinose est fait réagir avec un alcool X-OH, où X est tel que défini à la revendication 1, pour former ainsi le composè représenté par la formule S.

4. Procédé selon la revendication 2, dans lequel le dérivé dihydroxy représenté par la formule E est fait réagir avec p-toluenesulfonyl-chlorure dans la phase (c).
